(19) Europäisches Patentamt
European Patent Office
Office européen des brevets

(11) Publication number: **0 118 163 B1**

# (12) EUROPEAN PATENT SPECIFICATION

(45) Date of publication of patent specification: **20.04.88**

(51) Int. Cl.$^4$: **C 12 P 13/00**, A 01 N 37/38 // C12N9/18

(21) Application number: **84300024.1**

(22) Date of filing: **04.01.84**

## (54) Method for biotechnologically preparing optically active alpha-cyano-3-phenoxybenzyl alcohol.

(30) Priority: **10.01.83 JP 2602/83**
**10.01.83 JP 2603/83**
**10.01.83 JP 2604/83**

(43) Date of publication of application:
**12.09.84 Bulletin 84/37**

(45) Publication of the grant of the patent:
**20.04.88 Bulletin 88/16**

(84) Designated Contracting States:
**CH DE FR GB IT LI NL**

(56) References cited:
**EP-A-0 036 774**
**EP-A-0 080 827**
**FR-A-2 053 218**
**FR-A-2 295 933**
**FR-A-2 447 899**
**US-A-3 607 651**
**AGRICULTURAL & BIOLOGICAL CHEMISTRY, vol. 45, no. 6, June 1981, pages 1389-1392, Tokyo, JP S. IRIUCHIJIMA et al.: "Asymmetric hydrolysis of (+-)-alpha-substituted carboxylic acid esters with microorganisms"**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Mitsuda, Satoshi**
**10-4-439, Sonehigashimachi 2-chome**
**Toyonaka-shi Osaka 561 (JP)**
Inventor: **Hirohara, Hideo**
**4-31-18, Takakuradai**
**Sakai-shi Osaka 590-01 (JP)**

(74) Representative: **Myerscough, Philip Boyd et al**
**J.A.Kemp & Co. 14, South Square Gray's Inn**
**London, WC1R 5EU (GB)**

(56) References cited:
**PATENTS ABSTRACTS OF JAPAN, vol. 5, no. 111 (C-63) (783), 18th July 1981**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for biotechnologically preparing optically active α-cyano-3-phenoxybenzyl alcohol. More particularly, it relates to a method for preparing optically active α-cyano-3-phenoxybenzyl alcohol which is rich in (S)-isomer (hereinafter referred to as "(S)-rich alcohol"), characterized by that an ester of (R,S)-α-cyano-3-phenoxybenzyl alcohol of the formula [I]:

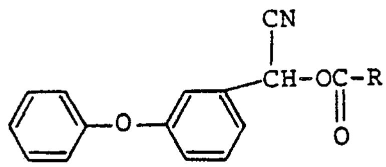

[I]

wherein R is alkoxy group having 1 to 8 carbon atoms, alkenyloxy group having 1 to 8 carbon atoms, alkynyloxy group having 1 to 8 carbon atoms, halogen substituted alkyl group having 1 to 4 carbon atoms, halogen substituted alkenyl group having 1 to 4 carbon atoms, halogen substituted alkynyl group having 1 to 4 carbon atoms, carboxyl group, hydroxycarbonylalkyl group having 2 to 4 carbon atoms which may be substituted with lower alkyl group or halogen atom, hydroxycarbonylalkenyl group having 3 to 4 carbon atoms which may be substituted with lower alkyl group or halogen atom, or hydroxycarbonylalkynyl group having 3 to 4 carbon atoms which may be substituted with lower alkyl group or halogen atom, is allowed to react with an esterase originated from a microorganism, an animal pancreas or liver, which is capable of asymmetrically hydrolyzing the ester of the formula [I], at pH not higher than 7 to asymmetrically hydrolyze the said ester to give the "(S)-rich alcohol".

α-Cyano-3-phenoxybenzyl alcohol has been known as a novel alcohol moiety of a series of ester compounds of synthetic pyrethroids, having superior insecticidal activity. The synthetic pyrethroids, typically fenvalerate, cypermethrin and deltamethrin, which have the said alcohol as their alcohol moiety, are good insecticides not only for household and sanitary uses but even for agricultural use, since they are resistant to sun-light and highly effective. Thus, the importance of this α-cyano-3-phenoxybenzyl alcohol is increasing.

Now, α-cyano-3-phenoxybenzyl alcohol has an asymmetric carbon at its α-position and has two optical isomers, i.e. (S)- and (R)-isomers. It has been known that the insecticidal activity of the ester from the (S)-isomer alcohol is far superior to that of any ester from the (R)-isomer alcohol. (see Hirosuke Yoshioka; Journal of Synthetic Organic Chemistry, Japan, Vol. 38, No. 12, pages 1151—1162, 1980). Therefore, the development of a commercially advantageous technology is desirable in an optical resolution of (R,S)-α-cyano-3-phenoxybenzyl alcohol, which is prepared according to the conventional method of synthetic chemistry, in order to obtain the (S)-isomer.

α-Cyano-3-phenoxybenzyl alcohol is an unstable compound and a conventional optical resolution process for an alcohol which is effected via an ester or a half-ester with an ordinary optically active organic acid may not be advantageous for it. As the methods to obtain (S)-α-cyano-3-phenoxybenzyl alcohol, the following methods have been known; (1) a method wherein desired (S)-α-cyano-3-phenoxybenzyl alcohol is obtained by reacting (R,S)-α-cyano-3-phenoxybenzyl alcohol with cis-2,2-dimethyl-3(S)-(dihydroxymethyl)-cyclopropane-1(R)-carboxylic acid lactone in the presence of an acidic reagent to obtain an ether compound, separating the resulting mixture of two kinds of the isomers each other according to a physical means, and hyrolysing the ether compound containing (S)-isomer alcohol component in an acidic medium (see Japanese Unexamined Published Patent Application No. 109944/1979); and (2) a method wherein (S)-α-cyano-3-phenoxybenzyl alcohol is obtained by reacting an (S)-α-cyano-3-phenoxybenzyl alcohol ester of a chiral cyclopropanecarboxylic acid with a boron halide and then with water (see Japanese Unexamined Published Patent Application No. 12355/1981).

These methods are complicated, need an expensive optically active reagent, and do not give a high overall yield. Furthermore, in method (2), the optically active α-cyano-3-phenoxybenzyl alcohol ester has to be prepared in advance. From these points, the methods heretofore known are not satisfactory.

After studies to overcome these difficulties and to seek a commercially advantageous method for preparing (S)-α-cyano-3-phenoxybenzyl alcohol, the present inventors have found that "(S)-rich alcohol" can be produced efficiently from an ester of the formula [I], as the starting material, and asymmetrically hydrolyzing it according to a biotechnological process.

Accordingly, the present invention provides a method for preparing the "(S)-rich alcohol", wherein an ester of the formula [I] is subjected to asymmetric hydrolysis using an esterase originated from a microorganism or an animal pancreas or liver, which is capable of asymmetrically hydrolyzing the ester of the (S)-α-cyano-3-phenoxybenzyl alcohol predominantly, at pH not higher than 7 to asymmetrically hydrolyze the ester, thereby to give the "(S)-rich alcohol" and the ester of (R)-α-cyano-3-phenoxybenzyl alcohol.

Hereunder, the present invention will be described in detail.

The term "esterase" used herein is of broad meaning including lipase having activity to a water-insoluble substrate. The esterases employable in the present invention are those capable of asymmetrically

hydrolyzing the ester of the (S)-α-cyano-3-phenoxybenzyl alcohol predominantly and they are originated from microorganisms and from animal pancreases or livers.

When the ester of formula [I] which is employed as the starting material in the present invention is a carbonic acid ester, i.e. when R is alkoxyl group having 1 to 8 carbon atoms, alkenyloxy group having 1 to 8 carbon atoms or alkynyloxy group having 1 to 8 carbon atoms, employable esterases are from microorganisms of genuses illustrated below: *Arthrobacter, Achromobacter, Pseudomonas, Chromobacterium, Bacillus, Brevibacterium, Nocardia, Rhodotorula, Candida, Trichoderma, Rhizopus, Mucor, Aspergillus, Geotricum, Aureobasidium, Hansenula, Torulopsis* and *Alcaligenes.*

When the ester of formula [I] is an ester of a halogen substituted organic carboxylic acid, i.e. when R is halogen substituted alkyl group having 1 to 4 carbon atoms, halogen substituted alkenyl group having 1 to 4 carbon atoms or halogen substituted alkynyl group having 1 to 4 carbon atoms, employable esterases are from an animal pancreas or liver or microorganisms of genuses illustrated below: *Arthrobacter, Pseudomonas, Chromobacterium, Bacillus, Candida, Nocardia, Rhodotorula, Brevibacterium, Trichoderma, Rhizopus, Mucor, Aspergillus, Geotricum, Hansenula, Enterobacter, Flavobacterium, Mycobacterium, Corynebacterium, Lactobacillus, Streptmyces, Penicillium, Actinomucor, Gibberalla, Absidia, Cunninghamella, Gliocladium, Saccharomyces, Cryptococcus, Pichia, Micrococcus, Torulopsis, Aureobasidium, Alcaligenes* and *Achromobacter.*

In these microorganisms, in particular, the microorganisms belonging to genuses: *Arthrobacter, Achromobacter, Pseudomonas, Chromobacterium, Bacillus, Brevibacterium, Nocardia, Rhodotorula, Candida, Trichoderma, Rhizopus, Mucor, Aspergillus, Geotricum, Aureobasidium, Hansenula* and *Torulopsis,* are preferred from viewpoints of optical selectivity and ability of hydrolysis.

When the ester of the formula [I] is a half-ester, i.e. when R is a carboxyl group, hydroxycarbonylalkyl group having 2 to 4 carbon atoms which may be substituted with lower alkyl group or halogen atom, hydroxycarbonyl alkenyl group having 3 to 4 carbon atoms which may be substituted with lower alkyl group or halogen atom, or hydroxycarbonyl alkynyl group having 3 to 4 carbon atoms which may be substituted with lower alkyl group or halogen atom, employable esterases are from microorganisms of genuses illustrated below: *Arthrobacter, Chromobacterium, Rhodotolura, Torulopsis, Hansenula* and *Candida.*

Examples of microorganisms described above are the strains mentioned below:

(1) *Arthrobacter simplex* IFO—3530
(2) *Achromobacter* sp. ATCC—21910
(3) *Pseudomonas fluorescens* IFO—3081
(4) *Chromobacterium viscosum* ATCC—6918
(5) *Bacillus licheniformis* IFO—12197
(6) *Brevibacterium ammoniagenes* IFO—12072
(7) *Nocardia erythropolis* IFO—12320
(8) *Rhodotorula minuta* var. *texensis* IFO—0879
(9) *Candida utilis* IFO—1086
(10) *Trichoderma viride* IFO—4847
(11) *Rhizopus chinensis* IFO—4737
(12) *Mucor javanicus* IFO—4572
(13) *Aspergillus* var. *asper* IFO—5324
(14) *Geotricum candidum* IFO—5368
(15) *Aureobasidium pullulans* IFO—4464
(16) *Hansenula anomala* IFO—0707
(17) *Torulopsis candida* IFO—0380
(18) *Alcaligenes faecalis* IFO—12669
(19) *Enterobacter cloacae* IFO—3320
(20) *Flavobacterium arborescens* IFO—3750
(21) *Mycobacterium phlei* IFO—3185
(22) *Corynebacterium equi* ATCC—7699
(23) *Pichia polimorpha* IFO—1166
(24) *Micrococcus luteus* IFO—3066
(25) *Cryptococcus albidus* IFO—0378
(26) *Lactobacillus casei* IFO—3322
(27) *Saccharomyces ruoxii* IFO—0505
(28) *Penicillium frequentans* IFO—5692
(29) *Actinomucur elegans* IFO—4022
(30) *Streptomyces griseus* IFO—3356
(31) *Gibberella zeae* IFO—7160
(32) *Absidia hyalospora* IFO—8082
(33) *Cunninghamella elegans* IFO—6334
(34) *Gliocladium roseum* IFO—5422

The above strains of the microorganisms have been deposited in American Type Culture Collection, U.S.A., (ATCC) or Institute for Fermentation, Osaka, Japan, (IFO) and are available from such depositories.

Some of the esterases originated from a microorganism or an animal pancreas or liver are commercially available and they can also be employed to fulfil the object of the present invention. The commercially available enzymes employable in the invention are illustrated below.

| Lipase | Origin | Seller or Manufacturer |
|---|---|---|
| Lipase AP | *Aspergillus* sp. | Amano Seiyaku |
| Lipase M-AP | *Mucor* sp. | " |
| Lipase "Amano" P | *Pseudomonas* sp. | " |
| Lipase Godo BSL | *Arthrobacter ureafaciens* nov. var | Godo Shusei |
| Lipase "Toyo" | *Chromobacterium viscosum* var. *paralipolyticum* | Toyo Jozo |
| Lipase "Saiken" | *Rhizopus* sp. | Osaka Saikin Kenkyujo |
| Lipase AL | *Achromobacter* sp. | Meito Sangyo |
| Lipase PL No.266 | *Alcaligenes* sp. | " |
| Lipase PL No.679 | *Alcaligenes* sp. | " |
| Steapsin | Hog pancreas | Tokyo Kasei |

The starting ester may easily be prepared according to the conventional esterification process. For example, the carbonic acid ester is prepared by reacting (R,S)-α-cyano-3-phenoxybenzyl alcohol with alkyl chlorocarbonate, e.g. methyl chlorocarbonate, ethyl chlorocarbonate or propyl chlorocarbonate. The ester (A) of a halogen substituted organic carboxylic acid is prepared by reacting (R,S)-α-cyano-3-phenoxybenzyl alcohol with an anhydride of a halide or such organic acid, preferably an organic acid having 1 to 2 halogen atoms on the α-position, more preferably an organic acid having a chlorine or bromine atom on the α-position, such as monochloroacetic acid or mono bromoacetic acid. Alternatively, the (A) may be prepared by reacting 3-phenoxybenzaldehyde and sodium cyanide with a halide of such organic carboxylic acid. The half-ester is prepared by reacting (R,S)-α-cyano-3-phenoxybenzyl alcohol with an anhydride of dicarboxylic acid, e.g. oxalic acid, malonic acid, succinic acid, glutaric acid, maleic acid, fumaric acid or a substitution product with lower alkyl group or halogen atom, preferably succinic acid from the viewpoint of ease in handling.

In practicing the method of the present invention, the asymmetric hydrolysis of the ester of the formula [I], is conducted by stirring or shaking vigourously a mixture of the said ester and an esterase-containing liquor, such as cultured liquor of such microorganism, its filtrate, esterase extracted liquor and its concentrate, suspension or microorganism cells or aqueous solution containing crude or purified esterase preparation or animal pancreas or liver esterase preparation. If required, surfactant of non-ester type may be added thereto. Alternatively, the enzyme may be employed in an immobilized state.

In this process, the reaction temperature is suitably 10° to 65°C. More preferable temperature is 20° to 50°C, since the resulting alcohol is comparatively unstable at a higher temperature and the reaction rate is low at a lower temperature.

The period of time for the reaction is normally 0.5 to 48 hours, but it can be shortened at a higher reaction temperature or by use of an enzyme of high activity. It is essential to control the pH of the aqueous solution during the asymmetric hydrolysis reaction, because α-cyano-3-phenoxybenzyl alcohol tends to decompose especially by a basic substance. In a basic medium, the resulting alcohol is subjected to decomposition, though the asymmetric hydrolysis proceeds well by esterase. Accordingly, the hydrolysis reaction should be conducted at not higher than pH 7. While, too low pH tends to cause the deactivation of enzymes. Thus, it is preferred that the reaction is conducted in the range of pH 3.5 to pH 6.3. Further, if necessary, a buffer solution may be used to prevent the lowering of pH value due to the formation of an organic acid during the course of hydrolysis. As the buffer solution, either inorganic or organic acid salt buffer may be employed.

The concentration of the ester employed as the substrate may be in the range of 1 to 80% (w/w), preferably 5 to 35% (w/w), of the reaction mixture.

Then, after the asymmetric hydrolysis reaction the "(S)-rich alcohol" and the unreacted ester of the antipode are separated from the reaction mixture. For that purpose, solvent extraction, liquid phase separation by standing, column chromatography or other procedures can be adequately employed. For example, the reaction mixture may be extracted with an organic solvent, such as ether, benzene or toluene, and the extract is subjected to a column chromatography using, for example, silica gel to isolate "(S)-rich alcohol".

On the other hand, the unreacted ester thus separated may be used again as the starting material of the method of the present invention after the epimerization by contact with a basic compound, such as ammonia, pyridine or triethylamine.

Hereunder, the present invention will be more fully described by means of the following Examples.

Examples 1 to 8

To 15 ml of 0.2 M concentration of an acetate buffer solution (pH 4.0) were added 2.0 g of (R,S)-α-cyano-3-phenoxybenzyl ethylcarbonate and each 40 mg of an esterase illustrated in Table 1. The mixture was stirred at 40°C for 24 hours. And then, the reaction mixture was extracted with toluene. The extract was analyzed by high-performance liquid chromatography (HPLC) (Lichrosorb RP-18, MeOH-water (6:4), 254 nm, UV detection), and the rate of the hydrolysis was calculated from the peak area ratio of unreacted α-cyano-3-phenoxybenzyl ethylcarbonate and free α-cyano-3-phenoxybenzyl alcohol. The toluene extract was concentrated, and subjected to a silica gel chromatography and eluted with cyclohexane-diethyl ether (95:5) mixture to isolate the unaltered ethylcarbonate of α-cyano-3-phenoxybenzyl alcohol, followed by being removed. The column was then eluted with methanol containing a trace ($10^{-5}$%) of p-toluenesulfonic acid, to obtain the solution of free α-cyano-3-phenoxybenzyl alcohol.

After removing the solvent from the elute, 10 mg of the obtained free α-cyano-3-phenoxybenzyl alcohol was dissolved in 1 ml of toluene, and reacted with the equal mole amount of (S)-(+)-2-(4-chlorophenyl)-isovaleric acid chloride in the presence of pyridine to convert it to a diastereomer of α-cyano-3-phenoxybenzyl alcohol (S)-(+)-2-(4-chlorophenyl)-isovalerate, which was analyzed for the optical isomer ratio by gas chromatography (10% DCQF-1, 2.5 m, 250°C).

The results are shown in Table 1.

TABLE 1

| Example No. | Origin of esterase (name of enzyme) | Rate of hydrolysis (%) | Optical isomer ratio of free α-cyano-3-phenoxybenzyl alcohol [(S)-isomer: (R)-isomer] |
|---|---|---|---|
| 1 | *Arthrobacter ureafaciens* nov. var. (Lipase Godo BSL) | 52.1 | 93.7:6.3 |
| 2 | *Chromobacterium* sp. (Lipase "Toyo") | 50.0 | 100:0 |
| 3 | *Pseudomonas* sp. (Lipase "Amano" P) | 10.2 | 89.3:10.7 |
| 4 | *Aspergillus* sp. (Lipase AP) | 14.9 | 83.3:16.7 |
| 5 | *Mucor* sp. (Lipase M-AP) | 9.5 | 62.4:37.6 |
| 6 | *Alcaligenes* sp. (Lipase PL-679) | 37.2 | 85.1:14.9 |
| 2 | *Achromobacter* sp. (Lipase AL) | 46.6 | 79.5:20.5 |
| 8 | *Rhizopus* sp. (Lipase "Saiken") | 6.3 | 94.1:5.9 |

Examples 9 to 14

In a 500 ml flask having shoulder was put 100 ml of liquid medium [a sugared bouillon medium (prepared by dissolving 10.0 g of glucose, 5.0 g of peptone, 5.0 g of meat extract and 3.0 g of NaCl in 1 l of water and adjusting to pH 7.2) for bacteria as in Examples 9 and 10, or a malt extract-yeast extract medium (prepared by dissolving 5.0 g of peptone, 10.0 g of glucose, 3.0 g of malt extract and 3.0 of yeast extract in 1 l of water, and adjusting to pH 6.5) for fungi and yeasts as in Examples 11 to 14. After sterilization, the medium was inoculated with 2 platinum loops of a slant cultured microorganism as illustrated in Table 2, and cultured on a reciprocating shaker at 30°C for 72 hours. The pH value of the cultured medium was adjusted to pH 4.5 by use of an aqueous 2 M HCl solution. To the cultured medium was added 3.0 g of (R,S)-α-cyano-3-phenoxybenzyl propylcarbonate, and the mixture was stirred at 30°C for 30 hours.

Thereafter, the rate of the hydrolysis and the optical isomer ratio of the free α-cyano-3-phenoxybenzyl alcohol obtained was calculated by the same procedures as in Examples 1 to 8.

The results are shown in Table 2.

TABLE 2

| Example No. | Origin of esterase (microorganism cultured) | Rate of hydrolysis (%) | Optical isomer ratio of free α-cyano-3-phenoxybenzyl alcohol [(S)-isomer: (R)-isomer] |
|---|---|---|---|
| 9 | *Nocardia erythropolis* IFO—12320 | 13.0 | 85.2:14.8 |
| 10 | *Bacillus sphaericus* IFO—3528 | 13.0 | 61.5:38.5 |
| 11 | *Rhotorula minuta* var. *texensis* IFO—0879 | 31.8 | 73.0:27.0 |
| 12 | *Torulopsis candida* IFO—0380 | 21.3 | 75.8:4.2 |
| 13 | *Candida utilis* IFO—1086 | 22.6 | 60.3:39.7 |
| 14 | *Hansenula anomala* IFO—0707 | 33.7 | 90.9:9.1 |

Examples 15 to 23

To 15 ml of 0.2 M concentration of acetate buffer solution (pH 4.0) were added 4.0 g of (R,S)-α-cyano-3-phenoxybenzyl monochloroacetate and 40 mg of an esterase illustrated in Table 3. The mixture was stirred at 40°C for 5 hours under controlling the pH value at a constant level using pH controller by addition of an aqueous 1 M NaOH solution in the form of finely divided water drops formed by a drop controller. Thereafter, the rate of the hydrolysis and the optical isomer ratio of the obtained free α-cyano-3-phenoxybenzyl alcohol were calculated by the same procedures as in Examples 1 to 8. The results are shown in Table 3.

TABLE 3

| Example No. | Origin of esterase (name of enzyme) | Rate of hydrolysis (%) | Optical isomer ratio of free α-cyano-3-phenoxybenzyl alcohol [(S)-isomer: (R)-isomer] |
|---|---|---|---|
| 15 | *Arthrobacter ureafaciens* nov. var. (Lipase Godo BSL) | 53.9 | 93.0:7.0 |
| 16 | *Chromobacterium* sp. (Lipase "Toyo") | 50.0 | 100:0 |
| 17 | *Pseudomonas* sp. (Lipase "Amano" P) | 49.5 | 93.9:6.1 |
| 18 | *Aspergillus* sp. (Lipase AP) | 70.3 | 63.4:36.6 |
| 19 | *Mucor* sp. (Lipase M-AP) | 28.3 | 83.8:16.2 |
| 20 | *Alcaligenes* sp. (Lipase PL-679) | 49.0 | 70.3:29.7 |
| 21 | *Achromobacter* sp. (Lipase AL) | 45.7 | 88.1:11.9 |
| 22 | *Rhizopus* sp. (Lipase "Saiken") | 24.6 | 89.9:10.1 |
| 23 | Hog pancreas (steapsin) | 35.1 | 79.4:20.6 |

Examples 24 to 28

In a 500 ml flask having shoulder was put 100 ml of malt extract—yeast extract medium which was prepared by the same procedures as in Examples 11 to 14. After sterilization, the medium was inoculated with 2 platinum loops of a slant cultured microorganism as illustrated in Table 4, and cultured on a reciprocating shaker at 30°C for 72 hours. The pH value of the cultured medium was adjusted to pH 4.5 by use of an aqueous 2 M HC solution. To the cultured medium was added 2.0 g of (R,S)-α-cyano-3-phenoxybenzyl monobromoacetate, and the mixture was stirred at 30°C for 15 hours while maintaining the pH value at a constant level in the similar way to Examples 15 to 23.

Thereafter, the rate of the hydrolysis and the optical isomer ratio of the obtained free α-cyano-3-phenoxybenzyl alcohol were calculated by the same procedures as in Examples 1 to 8. The results are shown in Table 4.

TABLE 4

| Example No. | Origin of esterase (microorganism cultured) | Rate of hydrolysis (%) | Optical isomer ratio of free α-cyano-3-phenoxybenzyl alcohol [(S)-isomer: (R)-isomer] |
|---|---|---|---|
| 24 | *Rhodotorula minuta* var. *texenis* IFO—0879 | 39.5 | 64.5:35.5 |
| 25 | *Torulopsis candida* IFO—0380 | 28.1 | 65.2:34.8 |
| 26 | *Candida utilis* IFO—1086 | 38.5 | 61.2:38.8 |
| 27 | *Hansenula anomala* IFO—0707 | 44.0 | 63.3:36.7 |
| 28 | *Trichoderma viride* | 31.0 | 70.1:29.9 |

Examples 29 to 30

To 20 ml of 0.2 M concentration of acetate buffer solution (pH 4.0) were added 2.5 g of (R,S)-α-cyano-3-phenoxybenzyl acid succinate and 50 mg of an esterase illustrated in Table 5. The mixture was stirred at 40°C for 24 hours while maintaining the pH value at a constant level in the same procedures as in Examples 15 to 23. Thereafter, the rate of the hydrolysis was calculated by the similar way to Examples 1 to 8. Then the toluene extract was concentrated and subjected to a silica gel chromatography and eluted with cyclohexane-diethyl ether (92:8) mixture to isolate the unaltered acid succinate of a α-cyano-3-phenoxybenzyl alcohol. Thereafter, a free α-cyano-3-phenoxybenzyl alcohol was obtained and analyzed by the same procedures as in Examples 1 to 8. The results are shown in Table 5.

TABLE 5

| Example No. | Origin of esterase (name of enzyme) | Rate of hydrolysis (%) | Optical isomer ratio of free α-cyano-3-phenoxybenzyl alcohol [(S)-isomer: (R)-isomer] |
|---|---|---|---|
| 29 | *Chromobacterium viscosum* (Lipase "Toyo") | 39.4 | 78.2:21.8 |
| 30 | *Arthrobacter ureafaciens* nov. var. (Lipase Godo BSL) | 20.7 | 62.5:37.5 |

Examples 31 to 34

The cultured medium of microorganism illustrated in Table 6 was prepared by the same procedures as in Examples 24 to 28. To the cultured medium was added 2.0 g of sodium (R,S)-α-cyano-3-phenoxybenzyl succinate and the mixture was stirred at 35°C for 30 hours while maintaining the pH value at a constant level in the same procedures as in Examples 15 to 23. Thereafter, the rate of hydrolysis and the optical isomer ratio of obtained free α-cyano-3-phenoxybenzyl alcohol were calculated in the similar way to Examples 29 to 30. The results are shown in Table 6.

TABLE 6

| Example No. | Origin of esterase (microorganism cultured) | Rate of hydrolysis (%) | Optical isomer ratio of free α-cyano-3-phenoxybenzyl alcohol [(S)-isomer: (R)-isomer] |
|---|---|---|---|
| 31 | *Rhodotorula minuta* var. *texenis* IFO—0879 | 23.8 | 62.3:37.7 |
| 32 | *Torulopsis candida* IFO—0380 | 11.3 | 77.1:23.9 |
| 33 | *Hansenula anomala* IFO—0707 | 11.4 | 82.6:17.4 |
| 34 | *Candida utilis* IFO—1086 | 11.3 | 58.2:41.8 |

**Claims**

1. A method for biotechnically preparing optically active α-cyano-3-phenoxybenzyl alcohol which is rich in (S)-isomer, characterised by reacting an (R,S)-α-cyano-3-phenoxybenzyl alcohol ester of formula [I]:

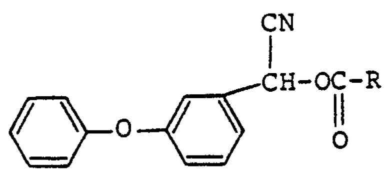

[I]

wherein R is alkoxyl group having 1 to 8 carbon atoms, alkenyloxy group having up to 8 carbon atoms, alkynyloxy group having up to 8 carbon atoms, halogen substituted alkyl group having 1 to 4 carbon atoms, halogen substituted alkenyl group having up to 4 carbon atoms, halogen substituted alkynyl group having up to 4 carbon atoms, carboxyl group, hydroxycarbonylalkyl group having 2 to 4 carbon atoms which may be substituted with a lower alkyl group or halogen atom, hydroxycarbonylalkenyl group having 3 to 4 carbon atoms which may be substituted with a lower alkyl group or halogen atom, or hydroxycarbonylalkynyl group having 3 to 4 carbon atoms which may be substituted with a lower alkyl group or halogen atom, with an esterase originating from a microorganism or an animal pancreas or liver and capable of asymmetrically hydrolyzing the ester of the formula [I], at pH not higher than 7 to asymmetrically hydrolyze the said ester to give the optically active α-cyano-3-phenoxybenzyl alcohol which is rich in (S)-isomer.

2. A method according to claim 1, wherein the ester of formula [I] is one in which R is an alkoxy group having 1 to 8 carbon atoms, an alkenyloxy group having up to 8 carbon atoms or an alkynyloxy group having up to 8 carbon atoms.

3. A method according to claim 1, wherein the ester of formula [I] is one in which R is an alkoxyl group having 1 to 8 carbon atoms.

4. A method according to claim 1, wherein the ester of formula [I] is one in which R is an alkoxyl group having 1 to 2 carbon atoms.

5. A method according to claim 1, wherein the ester of formula [I] is one in which R is a halogen-substituted alkenyl group having up to 4 carbon atoms or a halogen-substituted alkynyl group having up to 4 carbon atoms.

6. A method according to claim 1, wherein the ester of formula [I] is one in which R is an alkyl group having 1 to 2 halogen atoms on the α-position and 1 to 4 carbon atoms.

7. A method according to claim 1, wherein the ester of formula [I] is an ester of monochloroacetic acid, dichloroacetic acid or monobromoacetic acid.

8. A method according to claim 1, wherein the ester of formula [I] is a half-ester of oxalic acid, malonic acid, succinic acid, glutaric acid, maleic acid or fumaric acid.

9. A method according to claim 1, wherein any lower alkyl group present is of 1 to 4 carbon atoms.

10. Use of an (S)-rich alcohol prepared by a method as claimed in any one of the preceding claims in the preparation of a synthetic pyrethroid.

**Patentansprüche**

1. Ein Verfahren zur Herstellung auf biotechnologische Weise eines optisch aktiven α-Cyano-3-phenoxybenzylalkohols welcher reich ist am (S)-Isomeren, dadurch gekennzeichnet, daß ein (R,S)-α-Cyano-3-phenoxybenzylalkoholester der Formel (I):

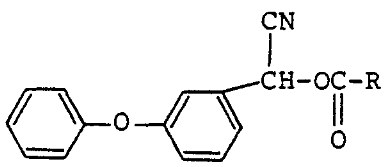

[I]

in welcher R eine Alkoxygruppe mit 1 bis 8 Kohlenstoffatomen, eine Alkenyloxygruppe mit bis zu 8 Kohlenstoffatomen, eine Alkinyloxygruppe mit bis zu 8 Kohlenstoffatomen, eine halogensubstituierte Alkylgruppe mit 1 bis Kohlenstoffatomen, eine halogensubstituierte Alkenylgruppe mit bis zu 4 Kohlenstoffatomen, eine halogensubstituierte Alkinylgruppe mit bis zu 4 Kohlenstoffatomen, eine Carboxylgruppe, eine Hydroxycarbonylgruppe mit 2 bis 4 Kohlenstoffatomen, welche substituiert sein kann mit einer niederen Alkylgruppe oder einem Halogenatom, eine Hydroxycarbonylalkenylgruppe mit 3 oder 4 Kohlenstoffatomen, welche substituiert sein kann mit einer niederen Alkylgruppe oder einem Halogenatom, oder eine Hydroxycarbonylalkinylgruppe mit 3 oder 4 Kohlenstoffatomen, welche mit einer niederen Alkylgruppe oder einem Halogenatom substituiert sein kann, darstellt, mit einer Esterase, welche von einem Mikroorganismus oder einer tierischen Bauchspeicheldrüse oder Leber stammt und den Ester der Formel (I) asymmetrisch hydrolysieren kann, bei einem pH-Wert von nicht höher als 7 zum asymmetrischen Hydrolysieren des genannten Esters umgesetzt wird, wobei dann der optisch aktive α-Cyano-3-phenoxybenzylalkohol, reich am (S)-Isomeren erhalten wird.

2. Ein Verfahren nach Anspruch 1, in welchem bei dem Ester der Formel (I) R eine Alkoxygruppe mit 1 bis 8 Kohlenstoffatomen, ein Alkenyloxygruppe mit bis zu 8 Kohlenstoffatomen oder eine Alkinyloxygruppe mit bis zu 8 Kohlenstoffatomen ist.

3. Ein Verfahren nach Anspruch 1, in welchem bei dem Ester der Formel (I) R eine Alkoxygruppe mit 1 bis 8 Kohlenstoffatomen ist.

4. Ein Verfahren nach Anspruch 1, in welchem bei dem Ester der Formel (I) R eine Alkoxygruppe mit 1 oder 2 Kohlenstoffatomen ist.

5. Ein Verfahren nach Anspruch 1, in welchem bei dem Ester der Formel (I) R eine halogensubstituierte Alkylgruppe mit 1 bis 4 Kohlenstoffatomen, eine halogensubstituierte Alkenylgruppe mit bis zu 4 Kohlenstoffatomen oder eine halogensubstituierte Alkinylgruppe mit bis zu 4 Kohlenstoffatomen ist.

6. Ein Verfahren nach Anspruch 1, in welchem bei dem Ester der Formel (I) R eine Alkylgruppe mit 1 oder 2 Halogenatomen in der α-Stellung und 1 bis 4 Kohlenstoffatomen ist.

7. Ein Verfahren nach Anspruch 1, in welchem bei dem Ester der Formel (I) ein Ester der Monochloressigsäure, der Dichloressigsäure oder der Monobromessigsäure ist.

8. Ein Verfahren nach Anspruch 1, in welchem bei dem Ester der Formel (I) ein Halbester der Oxalsäure, der Malonsäure, der Bernsteinsäure, der Glutarsäure, der Maleinsäure oder der Fumarsäure ist.

9. Ein Verfahren nach Anspruch 1, in welchem jede vorhandene niedere Alkylgruppe 1 bis 4 Kohlenstoffatomen hat.

10. Verwendung eines (S)-reichen Alkohols, hergestellt durch ein Verfahren wie in einem der vorstehenden Ansprüche beansprucht, für die Herstellung eines synthetischen Pyrethroids.

**Revendications**

1. Procédé de préparation par voie biotechnologique de l'alcool α-cyano-3-phénoxybenzylique optiquement actif et riche en isomère-(S), caractérisé en ce qu'il consiste à faire réagir un ester de l'alcool α-cyano-3-phénoxybenzylique-(RS), de formule (I):

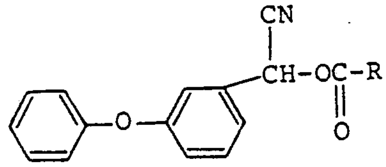

[I]

dans laquelle R est un groupe alcoxy de 1 à 8 atomes de carbone, un groupe alcényloxy ayant jusqu'à 8 atomes de carbone, un groupe alcynyloxy ayant jusqu'à 8 atomes de carbone, un groupe alcoyle de 1 à 4 atomes de carbone et substitué par un halogène, un groupe alcényle ayant jusqu'à 4 atomes de carbone et substitué par un halogène, un groupe alycnyle ayant jusqu'à 4 atomes de carbone et substitué par un

halogène, un groupe carboxyle, un groupe hydroxycarbonylalcoyle ayant de 2 à 4 atomes de carbone qui peut être substitué par un groupe alcoyle inférieur ou par un atome d'halogène, un groupe hydroxycarbonylalcényle ayant de 3 à 4 atomes de carbone qui peut être substitué par un groupe alcoyle inférieur ou par un atome d'halogène, ou un groupe hydroxycarbonylalcynyle ayant de 3 à 4 atomes de carbone qui peut être substitué par une groupe alcoyle inférieur ou part un atome d'halogène, sur une estérase provenant d'un microorganisme ou d'un pancréas ou d'un foie d'animal et susceptible d'hydrolyser asymètriquement l'ester de formula (I), à un pH qui n'est pas supérieur à 7, pour hydrolyser asymètriquement cet ester en l'alcool α-cyano-3-phénoxybenzylique optiquement actif qui est riche en l'isomère-(S).

2. Procédé suivant la revendication 1, dans lequel l'ester de formule (I) est un ester dans lequel R est un groupe alcoxy ayant de 1 à 8 atomes de carbone, un groupe alcényloxy ayant jusqu'à 8 atomes de carbone ou un groupe alcynyloxy ayant jusqu'à 8 atomes de carbone.

3. Procédé suivant la revendication 1, dans lequel l'ester de formule (I) est un ester dans lequel R est un groupe alcoxy ayant de 1 à 8 atomes de carbone.

4. Procédé suivant la revendication 1, dans lequel l'ester de formule (I) est un ester dans lequel R est un groupe alcoxy ayant de 1 ou 2 atomes de carbone.

5. Procédé suivant la revendication 1, dans lequel l'ester de formule (I) est un ester dans lequel R est un groupe alcoyle ayant de 1 à 4 atomes de carbone et substitué par un halogène, un groupe alcényle ayant jusqu'à 4 atomes de carbone et substitué par un halogène, ou un groupe alcynyle ayant jusqu'à 4 atomes de carbone et substitué par un halogène.

6. Procédé suivant la revendication 1, dans lequel l'ester de formule (I) est un ester dans lequel R est un groupe alcoyle ayant 1 ou 2 atomes d'halogène en la position α et ayant de 1 à 4 atomes de carbone.

7. Procédé suivant la revendication 1, dans lequel l'ester de formule (I) est un ester de l'acide monochloracétique, de l'acide dichloracétique ou de l'acide monobromacétique.

8. Procédé suivant la revendication 1, dans lequel l'ester de formule (I) est un hémi-ester de l'acide oxalique, de l'acide malonique, de l'acide succinique, de l'acide glutarique, de l'acide maléique ou de l'acide fumarique.

9. Procédé suivant la revendication 1, dans lequel tout groupe alcoyle inférieur présent a de 1 à 4 atomes de carbone.

10. Utilisation d'un alcool riche en (S), préparé par un procédé tel que revendiqué suivant l'une quelconque des revendications précédentes, pour la préparation d'un pyréthroïde synthétique.